# FASCICULE DE BREVET EUROPEEN

(11) **EP 4 040 139 B1**
(45) Date de publication et mention de la délivrance du brevet: **31.01.2024**
(21) Numéro de dépôt: 22154866.2
(22) Date de dépôt: 02.02.2022
(51) Int. Cl.: G01N 21/64, G01N 33/18, G01N 21/15, G01N 21/85

(54) **SONDE OPTIQUE POUR ANALYSE PAR FLUORESCENCE EN MILIEUX AQUEUX DE MOLÉCULES ORGANIQUES PRÉSENTANT AU MOINS UN FLUOROPHORE ET PROCÉDÉ DE MISE EN OEUVRE**
OPTISCHE SONDE ZUR FLUORESZENZANALYSE IN WÄSSRIGEN MEDIEN VON ORGANISCHEN MOLEKÜLEN MIT MINDESTENS EINEM FLUOROPHOR UND IHR ANWENDUNGSVERFAHREN
OPTICAL PROBE FOR ANALYSIS BY FLUORESCENCE IN AQUEOUS MEDIA OF ORGANIC MOLECULES HAVING AT LEAST ONE FLUOROPHORE AND METHOD FOR USING SAME

(30) Priorité: 05.02.2021 FR 2101142
(43) Date de publication de la demande: 10.08.2022
(73) Titulaire: SIAAP, 75589 Paris (FR); Université Paris-Est Créteil Val de Marne, 94010 Creteil Cedex (FR)
(72) Inventeur: ROCHER, Vincent, 78570 Andresy (FR); VARRAULT, Gilles, 94010 Créteil Cedex (FR); GOFFIN, Angélique, 77165 Cuisy (FR); GUERIN, Sabrina, 95230 Soisy-Sous-Montmorency (FR)
(74) Mandataire: Corret, Hélène

(56) Documents cités:
- DE-A1- 19 617 106
- JP-A- 2003 250 752
- KR-A- 20090 081 998
- US-A1- 2003 062 485
- US-A1- 2006 121 614

## Description

La présente invention appartient au domaine technique de l'analyse des molécules organiques en milieux aqueux.

Cette analyse est notamment utile pour caractériser la matière organique présente dans les milieux aquatiques, les stations de traitement des eaux usées et les stations de potabilisation.

En effet, la matière organique est impliquée dans un grand nombre de processus biogéochimiques.

Ainsi, dans le domaine de la potabilisation des eaux, si la matière organique est présente en trop grande quantité, elle peut être difficile à éliminer. Elle peut alors conduire à la formation de sous-produits de désinfection cancérigènes ou à une reviviscence bactérienne dans les canalisations qui acheminent l'eau traitée vers les consommateurs, avec des conséquences néfastes sur leur santé.

Dans le domaine du traitement des eaux usées, l'objectif est l'élimination de la matière organique par différents procédés épuratoires. Or, la connaissance de la quantité et de la nature de la matière organique présente peut permettre l'optimisation des procédés épuratoires et ainsi, une économie d'énergie et de réactifs.

Quelques paramètres physico-chimiques de l'eau sont très fréquemment utilisés pour caractériser la matière organique présente en solution dans un milieu aqueux : la demande chimique en oxygène (DCO), la demande biochimique en oxygène à 5 jours (DBOs) et la concentration en carbone organique dissous (COD).

Plusieurs méthodes analytiques permettant de déterminer ces paramètres sont connues.

La DBOs, la DCO et le COD sont déterminés par des méthodes analytiques de laboratoire classiques normalisées. Ces méthodes nécessitent le prélèvement de l'échantillon, éventuellement sa filtration puis l'analyse en elle-même. L'analyse dure de quelques dizaines de minutes à 5 jours. On peut citer, par exemple, pour la mesure de la DBO₅ la norme française iso 5815-1 :2019, et pour la mesure de la DCO, la norme française iso 6060 :1989.

Cependant, elles nécessitent des prélèvements ponctuels dont l'analyse demande ensuite plusieurs heures, voire plusieurs jours.

On connaît également des dispositifs de mesure qui permettent de détecter in situ et en temps réel certains fluorophores dans un milieu aqueux. Cependant, leur utilisation peut être limitée à un seul fluorophore, alors que la connaissance d'un nombre important de fluorophores est nécessaire pour déterminer les paramètres analytiques pertinents.

De surcroît, ces dispositifs différents sont souvent conçus pour détecter les mêmes fluorophores, au nombre de 3 ou 4 maximum, si bien que la répétition des mesures avec plusieurs dispositifs différents ne permet pas d'accéder à un nombre plus important de fluorophores.

On peut notamment citer le document DE19617106 A1 qui décrit une sonde de mesure spectrométrique à fluorescence qui est destinée à être placée dans le sol ou dans un milieu aqueux. Elle comprend un filtre associé à une seule source lumineuse d'excitation et un filtre associé à la détection des émissions fluorescentes, si bien qu'un seul fluorophore peut être détecté.

Le document US2006/0121614 A1 décrit une sonde optique destinée à être immergée dans un milieu aqueux. Elle comprend plusieurs canaux d'excitation (canaux transmettant vers le milieu des photons d'excitation à partir d'une source de photons) et plusieurs canaux d'émission (canal de collecte et de traitement des photons émis par les fluorophores dans le milieu en réponse à l'excitation reçue par les canaux d'excitation). Chaque canal d'excitation comprend une diode émettrice de lumière et un filtre, et chaque canal d'émission comprend un filtre et un détecteur. Cette sonde permet donc la détection de plusieurs fluorophores correspondant au nombre de canaux d'émission et donc de détecteurs.

Le document US2003/0062485 A1 décrit un fluoromètre destiné à analyser des échantillons placés dans un réceptacle.

Le document JP2003250752 A décrit une sonde du type endoscope pour réaliser des mesures sur des tissus vivants. Elle comporte une partie flexible comportant les moyens de transport de la lumière d'excitation ou d'émission et destinée à être introduite dans un corps humain, ainsi qu'un appareil de traitement comportant une source de lumière blanche, une roue à filtres, des moyens de traitement du signal d'émission et un système de contrôle.

Le document KR 20090081998 A décrit une sonde de mesure à fluorescence comprenant un boîtier pour des sources lumineuses d'excitation et un détecteur optique. Ce ee- boîtier délimite, dans sa partie inférieure, une partie conique pour le positionnement d'un échantillon.

L'objectif de l'invention est de proposer un dispositif amélioré permettant de caractériser *in situ* et en temps réel la nature et la concentration de la matière organique présente dans un milieu aqueux donné, et en particulier d'identifier différentes familles de molécules organiques (ex : protéines, substances humiques, etc.). Cela permettra d'optimiser l'élimination de la matière organique tout en limitant l'utilisation d'énergie et de réactifs que ce soit en épuration des eaux usées ou en potabilisation des eaux.

L'invention concerne une sonde optique telle que définie dans la revendication 1 destinée à être, en utilisation, au moins partiellement immergée dans un milieu aqueux comprenant des molécules organiques présentant au moins un fluorophore, comprenant :
- Un ensemble de n sources d'émission de lumière UV/visible à des longueurs d'onde différentes, n étant un entier supérieur ou égal à 2, lesdites sources étant agencées pour diriger de la lumière vers l'extérieur de la sonde et dans le milieu aqueux lorsque la sonde est en utilisation, lesdites sources étant associées à des premiers moyens de sélection,
- Un ensemble de m premiers filtres passe-bande différents, m étant un entier supérieur ou égal à 2, associé à des deuxièmes moyens de sélection agencé pour recevoir des émissions de fluorescence émises par ledit au moins un fluorophore présent dans le milieu aqueux en réponse à une excitation lumineuse provenant de l'une desdites sources d'émission de lumière ;
- Un unique détecteur d'émissions de fluorescence filtrées par un premier filtre sélectionné par lesdits deuxièmes moyens de sélection associés audit ensemble de m premiers filtres et transmises depuis ledit ensemble , pour convertir lesdites émissions en un signal électrique et
- Une unité centrale conçue pour contrôler les premiers et deuxièmes moyens de sélection et pour déterminer la concentration dudit au moins un fluorophore, à partir dudit signal électrique.

Cette sonde peut être directement placée dans un milieu aqueux à analyser, qu'il s'agisse d'un milieu aquatique continental (rivière, lac, eaux souterraines par exemple), d'un milieu aquatique marin ou en usines d'épuration des eaux usées ou de potabilisation, dès lors qu'il est nécessaire de quantifier la matière organique en distinguant différentes familles de matière organique.

De manière plus précise, peuvent être cités :
- un réseau de collecte des eaux usées séparatif et unitaire
- le traitement des eaux usées
- le suivi de la qualité des milieux récepteurs (rivières, plans d'eau, milieu marin)
- le suivi des hydrocarbures en milieu marin
- le suivi des rejets d'eaux usées dans les milieux récepteurs
- la potabilisation des eaux (procédés de production d'eau potable, eau de nappes souterraines, réseau d'adduction)
- le domaine agro-alimentaire
- tout procédé de production industrielle comprenant des produits/réactifs présentant des propriétés de fluorescence.

Cette sonde met en oeuvre le principe de fluorescence qui permet de réaliser des analyses avec une très bonne sensibilité. Il convient de noter ici que la sonde ne comportant qu'un seul détecteur d'émissions de fluorescence, il est possible de choisir un détecteur très performant, sans conséquence défavorable sur le coût ou l'encombrement de la sonde. Cette dernière est basée sur le principe suivant : lorsqu'une molécule organique présentant un ou plusieurs groupements fluorophores (groupement d'atomes capable de réémettre de la lumière lorsqu'il reçoit une lumière d'excitation, ci-après dénommés par simplification fluorophores) absorbe un photon de longueur d'onde comprise entre 275 et 600 nm et correspondant à l'un de ces fluorophores, elle réémet un photon de longueur d'onde plus importante comprise entre 300 et 700 nm. L'intensité de la fluorescence peut être directement proportionnelle à la concentration du fluorophore.

Ainsi, à chaque fluorophore peut être associé un couple de paramètres (longueur d'onde d'excitation/longueur d'onde d'émission fluorescence).

Cette sonde permet, à elle seule, l'analyse d'au moins 10 fluorophores différents et, de préférence, de plusieurs dizaines de fluorophores différents, par exemple 20, 30, 40 ou plus, et donc l'analyse de plusieurs familles de molécules organiques, avec une haute sensibilité, ce qui permet de mieux caractériser la matière organique dissoute et de déterminer avec une grande fiabilité notamment trois paramètres classiquement étudiés (DCO, DBOs et COD). En effet, le nombre de fluorophores que la sonde permet d'analyser est égal n multiplié par m, n étant le nombre de sources d'émission de lumière et m le nombre de premiers filtres passe-bande.

Il a, en effet, été montré en laboratoire que l'analyse des échantillons par spectroscopie de fluorescence pouvait fournir des indicateurs corrélés de manière statistiquement significative avec les valeurs de ces trois paramètres, mesurées par des méthodes de référence (Thèse « Potentiel d'utilisation de la spectrofluorimétrie 3D pour la caractérisation en ligne de la matière organique dissoute : de la station d'épuration au milieu récepteur. Ingénierie de l'environnement », Angélique Goffin. Université Paris-Est, 2017. Français).

La sonde évite donc l'acquisition et la manipulation de plusieurs dispositifs classiques différents et permet, de plus, d'obtenir beaucoup plus d'informations que ne le permettraient ces dispositifs qui sont intrinsèquement limités à la détection de seulement quelques fluorophores différents.

Par ailleurs, les analyses effectuées par la sonde sont facilement adaptées au milieu aqueux en cause et aux fluorophores recherchés, grâce à une programmation appropriée de l'unité centrale. Les plusieurs dizaines de fluorophores qui peuvent être mesurés accroissent considérablement le spectre de recherche de cette sonde. Par ailleurs, cette analyse est réalisée *in situ,* en temps réel et sans employer de réactifs.

La sonde présente également l'avantage d'être compacte, facilement transportable et donc d'une manipulation aisée pour un utilisateur.

- La sonde comprend un premier module et un deuxième module reliés entre eux par des moyens de connexion électriques et optiques, le premier module regroupant l'ensemble des sources d'émission de lumière, les premiers moyens de sélection et des moyens de collection optique conçus pour collecter des émissions de fluorescence émises par le milieu aqueux en réponse à l'excitation lumineuse provenant de l'une des dites sources d'émission de lumière et les transmettre au deuxième module par les moyens de connexion optiques, et le deuxième module regroupant l'ensemble de premiers filtres passe-bande, les deuxièmes moyens de sélection, le détecteur et l'unité centrale, le premier module étant destiné, en utilisation, à être immergé dans le milieu aqueux.
   Grâce à cette réalisation en deux modules, la sonde est plus robuste, les pièces les plus fragiles étant placées dans un module qui peut rester à l'extérieur du milieu aqueux et immobile.
- La sonde peut comprendre un amplificateur associé au détecteur.
- Le détecteur peut être placé dans le deuxième module, entre l'ensemble de filtres et l'unité centrale, comme l'éventuel amplificateur.
- Un deuxième filtre passe-bande peut être placé en aval de chacune des n sources d'émission de lumière.
- Des moyens de focalisation peuvent être prévus en amont des moyens de collection optique.
- Les deuxièmes moyens de sélection peuvent consister en une roue à filtres.
- L'unité centrale peut comprendre un microprocesseur, un dispositif de pilotage et une mémoire.
- La sonde peut comprendre un dispositif de contrôle de la dérive du fonctionnement des sources d'émission de lumière et du détecteur muni de l'éventuel amplificateur.
- La sonde peut comprendre un boîtier dont une partie formant hublot est transparente pour le passage de la lumière émise par les sources et des émissions de fluorescence émises par ledit au moins un fluorophore, en utilisation, dans le domaine UV/visible.
- La sonde peut comprendre un système de nettoyage associé audit hublot.
- Le dispositif de contrôle de la dérive du fonctionnement des sources d'émission de lumière peut comporter une lame réfléchissante.
- La lame réfléchissante peut être associée au système de nettoyage.

L'invention concerne également un procédé de mesure d'au moins un fluorophore dans un milieu aqueux comprenant des molécules organiques présentant au moins un fluorophore, le procédé utlilisant la sonde de l'invention et comprenant les étapes suivantes :
- La sélection d'une source d'émission de lumière parmi l'ensemble des n sources d'émission de lumière à des longueurs d'onde différentes, dans la gamme 250-600 nm ;
- L'émission de lumière à travers ledit milieu aqueux à partir de la source sélectionnée ;
- La sélection d'un filtre passe-bande parmi l'ensemble des premiers filtres ;
- La filtration et la détection des émissions de fluorescence émises par le milieu aqueux en réponse à l'excitation lumineuse provenant de ladite source sélectionnée ;
- Le traitement des émissions de fluorescence filtrées et détectées afin de déterminer la concentration dudit au moins un fluorophore.

Dans des modes de mise en oeuvre avantageux, on a par ailleurs et/ou de plus recours à l'une et/ou à l'autre des dispositions suivantes :
- La filtration des émissions de fluorescence peut être réalisée avant leur détection.
- Les émissions de fluorescence filtrées peuvent passer à travers un amplificateur avant d'être détectées et traitées.

L'invention sera mieux comprise et d'autres buts, avantages et caractéristiques de celle-ci apparaîtront plus clairement de la description qui suit de modes de réalisation donnés à titre d'exemples non limitatifs et qui est faite au regard des dessins annexés sur lesquels :
[Fig. 1], une vue schématique illustrant un premier mode de réalisation de la sonde non couvert par les revendications ;
[Fig. 2], une vue schématique illustrant un mode de réalisation de la sonde selon l'invention, en deux modules ;
[Fig. 3], une vue schématique partielle illustrant une première version de l'un des modules de la figure 2, avec un système de nettoyage en position escamotée et un dispositif de contrôle de la dérive inactif ;
[Fig. 4], une vue schématique en perspective illustrant le système de nettoyage dans la position escamotée de la figure 3 et le dispositif de contrôle de la dérive inactif ;
[Fig. 5], une vue schématique partielle illustrant le module de la figure 4, avec un dispositif de contrôle de la dérive actif ;
[Fig. 6], une vue schématique en perspective illustrant le dispositif de contrôle de la dérive actif ;
[Fig. 7], une vue schématique partielle illustrant une deuxième version de l'un des modules de la figure 2, avec un système de nettoyage en position escamotée et un dispositif de contrôle de la dérive inactif ;
[Fig. 8], une vue schématique de face et depuis l'extérieur du module de la figure 7 dont le système de nettoyage est en position escamotée et le dispositif de contrôle de la dérive est inactif ;
[Fig. 9], une vue schématique de face et depuis l'intérieur du module de la figure 7 dont le système de nettoyage est en position escamotée et le dispositif de contrôle de la dérive est inactif ;
[Fig. 10], une vue schématique partielle illustrant la deuxième version de l'un des modules de la figure 2, avec un dispositif de contrôle de la dérive en position active ;
[Fig. 11], une vue schématique de face et depuis l'extérieur du module de la figure 10 dont le dispositif de contrôle de la dérive est actif ; et
[Fig. 12], une vue schématique de face et depuis l'intérieur du module de la figure 10 dont le dispositif de contrôle de la dérive est actif.

Les éléments communs aux figures seront désignés par les mêmes références.

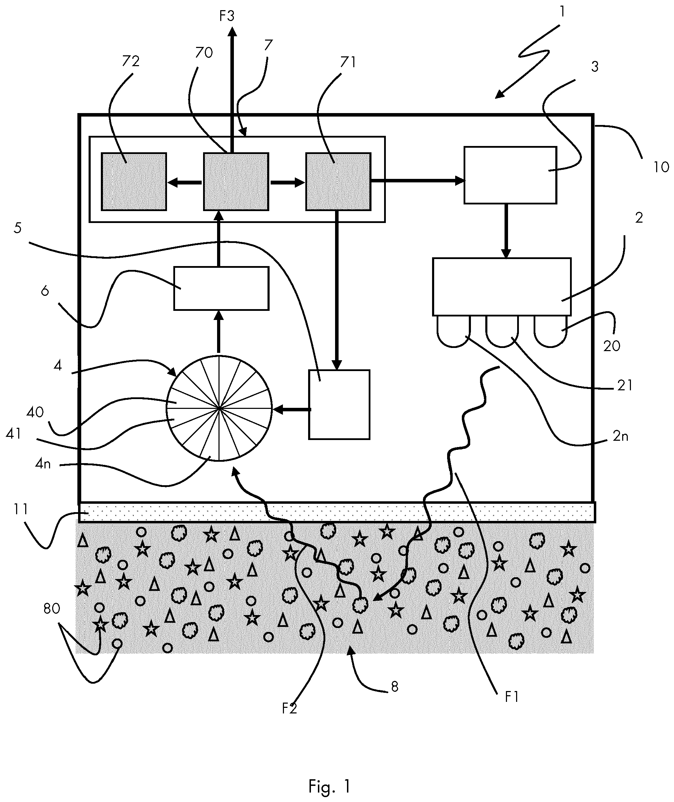

La figure 1 illustre une sonde 1 réalisée en un seul module.

Elle comporte un boîtier 10, par exemple de forme parallélépipédique, dont une face comporte une fenêtre ou un hublot 11 transparents et à travers lequel la source sélectionnée peut émettre des photons dans le domaine UV/visible vers l'extérieur du boîtier, dans le milieu aqueux 8.

À l'intérieur de ce boîtier, est prévu un ensemble 2 de n (n supérieur ou égal à 2) sources d'émission de lumière UV/visible (sources d'excitation). De préférence, le boîtier comporte au moins quatre sources d'excitation qui émettent à des longueurs d'onde différentes situées dans la gamme 250-600 nm, ou dans la gamme 250-400 nm ou encore dans la gamme 250-365 nm.

Bien entendu, ceci n'exclut pas que l'ensemble 2 comporte au moins deux sources émettant de la lumière UV/visible à la même longueur d'onde. Il peut, en effet, s'avérer nécessaire de faire fonctionner en même temps plusieurs sources émettant à la même longueur d'onde pour augmenter la sensibilité de la sonde.

La figure 1 illustre trois sources 20, 21,2n qui peuvent être par exemple des diodes électroluminescentes (ou LED) et émettre aux longueurs d'onde suivantes : 275 nm, 310 nm, 365 nm.

Ce boîtier comporte également des premiers moyens de sélection 3, reliés à l'ensemble 2 et grâce auxquels l'une de ces sources est sélectionnée, en fonction du fluorophore que l'on souhaite analyser dans le milieu aqueux.

Le boîtier comporte un ensemble 4 de m premiers filtres passe-bande (m supérieur ou égal à 2) différents reliés à des deuxièmes moyens de sélection 5 grâce auxquels un filtre donné peut être choisi, là encore en fonction du fluorophore que l'on souhaite analyser. Les longueurs d'onde correspondant à chaque filtre sont typiquement comprises entre 300 et 700 nm.

Cet ensemble de m premiers filtres passe-bande est agencé pour recevoir des émissions de fluorescence émises par des fluorophores présents dans le milieu aqueux en réponse à une excitation lumineuse provenant de l'une desdites sources d'émission de lumière. En d'autres termes, les m premiers filtres passe-bande sont en aval du milieu et reçoivent le rayonnement d'émission des fluorophores présents dans le milieu.

Chaque premier filtre présente une plage de longueurs d'onde qui s'étend de plus ou moins 10 mm autour de la longueur d'onde centrale du filtre où la transmittance est la plus importante.

Cet ensemble peut notamment se présenter sous la forme d'une roue à filtres 40, 41, 4n, par exemple commercialisée par la société Edmund Optics.

On pourrait également envisager de réaliser cet ensemble sous la forme d'une juxtaposition de filtres statiques et d'un miroir rotatif apte à diriger sélectivement les émissions de fluorescence provenant du milieu aquatique vers tel ou tel filtre statique.

Alternativement, on pourrait également envisager de réaliser cet ensemble sous la forme d'un râtelier de filtres combiné à un bras apte à diriger l'un des filtres du râtelier au travers des émissions de fluorescence provenant du milieu aquatique et à ranger le filtre en fin de mesure.

De façon générale, cet ensemble comporte au moins deux filtres passe-bande différents, c'est-à-dire laissant passer des photons de longueurs d'onde différentes et, de préférence au moins douze filtres différents.

Un détecteur 6 d'émissions de fluorescence et une unité centrale 7 sont également prévus dans le boîtier 1.

Le détecteur 6 est situé entre l'ensemble 4 de filtres et l'unité centrale 7 et reçoit les émissions de fluorescence filtrées transmises par l'ensemble de m premiers filtres.

La sonde selon l'invention comporte un unique détecteur pour toutes les émissions de fluorescence filtrées. Ceci distingue la sonde d'une sonde classique dans laquelle un détecteur spécifique est associé à chaque filtre et donc à un seul fluorophore. La conception de la sonde est ainsi simplifiée et la fiabilité améliorée.

L'unité centrale comporte par exemple un microprocesseur 70, un dispositif de pilotage 71 et une mémoire 72, le dispositif 71 et la mémoire 72 étant contrôlés par le microprocesseur 70.

Un amplificateur (non illustré sur la figure 1) peut être prévu entre l'ensemble 4 de filtres et le détecteur 6.

Comme l'illustre la figure 1, les premiers et deuxièmes moyens de sélection 3, 5 sont reliés au dispositif de pilotage 71 qui les contrôle.

L'ensemble 4 de filtres et les premiers et deuxièmes moyens de sélection 3, 5 sont reliés à une alimentation électrique (non illustrée sur la figure 1) qui est contrôlée par l'unité centrale 7.

De préférence, en aval de chaque source 20,21,2n, et en amont du hublot 11, c'est-à-dire entre ces sources et le hublot, est prévu un deuxième filtre passe-bande 50. Chacun de ces deuxièmes filtres 50 en aval d'une source reçoit donc le rayonnement d'excitation de cette source et permet de limiter la largeur de la raie d'excitation.

Cette sonde est au moins partiellement immergée dans le milieu aqueux 8, dans lequel se trouvent, sous forme dissoute, diverses molécules organiques 80, de manière à ce que le hublot soit, lui, totalement immergé dans le milieu aqueux 8.

Le fonctionnement de cette sonde, dans un milieu aqueux donné, est le suivant :

En fonction du fluorophore que l'on souhaite analyser dans le milieu 8, le microprocesseur 70 de l'unité centrale 7 commande, par l'intermédiaire du dispositif de pilotage 71, les premiers moyens de sélection 3 pour sélectionner la source lumineuse 20-21-2n émettant à la longueur d'onde souhaitée et les deuxièmes moyens de sélection 5 pour sélectionner le filtre passe-bande 40-41-4n approprié.

La source d'émission de lumière sélectionnée, par exemple la source 20, émet alors de la lumière à travers le milieu aqueux 8, de préférence filtrée par un filtre passe-bande. L'excitation lumineuse provenant de cette source est schématisée par la flèche F1.

Le fluorophore à analyser qui est présent dans le milieu aqueux 8 émet des émissions de fluorescence en direction du boîtier, en réponse à l'excitation lumineuse provenant de la source 20 sélectionnée. Les émissions de fluorescence, provenant du milieu 8 et traversant le hublot 10, sont schématisées par la flèche F2. Ces émissions sont dans le domaine UV/visible.

Les photons émis par fluorescence sont, dans un premier temps, filtrés par le filtre de l'ensemble 4 qui a été sélectionné, puis transmis au détecteur 6 pour être détectés et convertis en un signal électrique . Cette détection consiste à mesurer le nombre de photons reçus à la longueur d'onde sélectionnée et à le transformer en un signal électrique correspondant, éventuellement amplifié.

Ainsi, les photons émis par fluorescence sont filtrés, détectés et convertis en un signal électrique, signal qui sera ensuite traité par l'unité centrale 7 et, plus particulièrement, par le microprocesseur 70 auquel le détecteur 6 est relié.

Ce traitement réalisé par le microprocesseur permet de déterminer une intensité de fluorescence associée à un fluorophore (par exemple la tyrosine) ou à une famille de fluorophores (par exemple les acides humiques) qui est présent dans le milieu 8, à partir du signal fourni par le détecteur et généré en fonction du nombre de photons, cette intensité étant corrélée à une concentration du fluorophore ou de la famille de fluorophores. Par simplification, on parlera ensuite seulement de fluorophores.

Cette information peut être stockée dans la mémoire 72. Elle peut également être transmise par le microprocesseur 70 vers un dispositif d'affichage à destination de l'utilisateur de la sonde. Cette transmission est schématisée par la flèche F3.

On comprend donc que, grâce à la pluralité de sources lumineuses (n) et de filtres (m) présents dans la sonde, celle-ci est capable de détecter également une pluralité de fluorophores différents (nxm). Ainsi, une même sonde peut être utilisée pour tous ces fluorophores. Il suffit, avant une mesure, de choisir la source d'émission de lumière et le filtre adaptés à la détection d'un fluorophore donné, au moyen de l'unité centrale 7. Ce choix peut par exemple être permis à l'utilisateur grâce à un écran tactile muni de touches, chacune d'elles correspondant à un fluorophore.

À titre d'exemple, si la sonde comporte quatre sources d'émission de lumière et douze filtres passe-bande, elle permet l'analyse de 48 fluorophores différents, avec la même sonde, et dans un volume contenu.

En effet, la sonde reste compacte grâce à l'utilisation d'un unique détecteur pour la détection de toutes les émissions de fluorescence et d'un ensemble de filtres associé à des moyens de sélection. La sonde évite ainsi la présence d'un canal d'émission spécifique à chaque fluorophore, comprenant un détecteur dédié.

Ceci constitue un avantage considérable par rapport aux sondes connues qui, au contraire, comporte un canal d'émission dédié à chaque fluorophore, ce qui nécessite de multiplier les détecteurs.

La sonde selon l'invention permet d'analyser un très grand nombre de fluorophores, et de sélectionner parmi ceux-ci, les fluorophores les plus pertinents en fonction du site de la mesure et, d'une manière plus générale, de l'application envisagée.

La sonde selon l'invention est donc très polyvalente et convient pour un très grand nombre de conditions opérationnelles.

Cependant, en cas de nécessité, grâce à sa conception, il est facile d'adapter la sonde à des conditions d'analyses très particulières. En effet, si les fluorophores mesurables par la sonde ne conviennent pas à cette application, il est très facile de modifier les filtres de la roue à filtre, en particulier lorsque cette dernière est, dans un mode de réalisation préféré, dans un module émergé. Ainsi, la modification des filtres ne nécessite aucun réalignement de l'optique de la sonde, ni aucune modification de la sonde, notamment par ajout de détecteurs, et on aboutit à une sonde parfaitement adaptée à l'application envisagée.

Là encore, cet avantage est obtenu grâce à l'utilisation d'un unique détecteur d'émission de fluorescence et d'un ensemble de filtres associé à des moyens de sélection, de façon à sélectionner un filtre correspondant à un fluorophore.

Il apparaît que l'ensemble de filtres placés sur un ensemble motorisé 4, le détecteur 6 et/ou l'amplificateur sont des pièces électroniques fragiles qui peuvent être endommagées lorsque le boîtier est utilisé, du fait des vibrations et chocs potentiels.

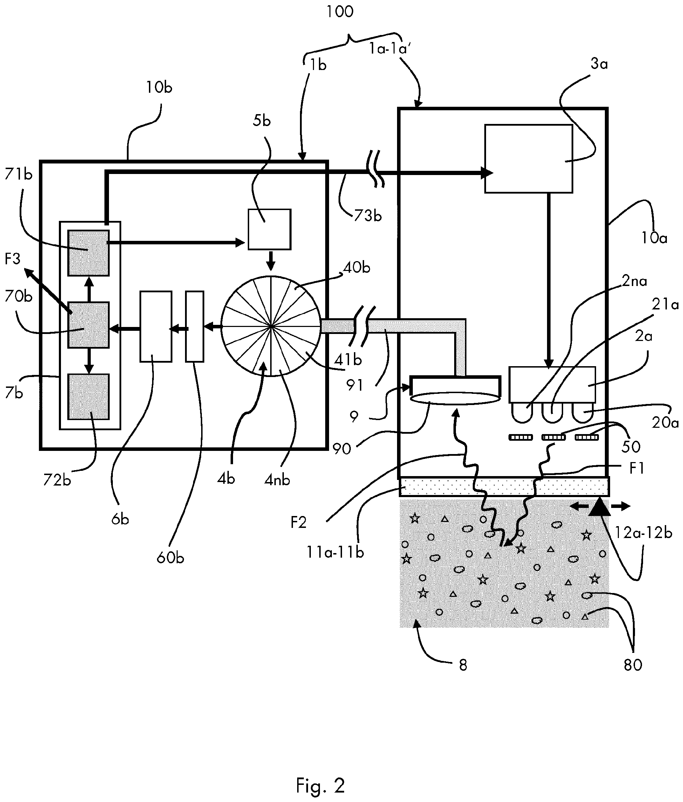

La figure 2 illustre une sonde selon l'invention conçue pour pallier cet inconvénient.

Cette sonde 100 est réalisée en deux modules distincts reliés électriquement et optiquement entre eux : un premier module 1a-1a' destiné à être immergé dans le milieu aqueux à analyser et un deuxième module 1b destiné à rester en surface et comprenant les éléments les plus fragiles.

Chacun de ces modules comporte un boîtier 10a, respectivement 10b, par exemple de forme parallélépipédique. Le boîtier 10a comporte une face avec une fenêtre ou un hublot 11a-11b transparents au rayonnement UV/visible.

Ainsi, le premier module 1a comporte un ensemble 2a de n (n supérieur ou égal à 2) sources d'émission de lumière UV 20a,21a,2na et de préférence au moins quatre sources qui émettent à des longueurs d'onde différentes situées dans la gamme 250-600 nm ou encore dans la gamme 275-365 nm.

II comporte des premiers moyens de sélection 3a, reliés à l'ensemble 2a, et qui permettent la sélection d'une de ces sources.

Ce premier module comporte également des moyens de collection optique 9 qui peuvent éventuellement comporter des moyens de focalisation 90. Ces derniers sont situés en amont des moyens de collection 9, c'est-à-dire avant ceux-ci sur le trajet des émissions de fluorescence.

Le deuxième module 1b comporte quant à lui un ensemble 4b de m premiers filtres passe-bande (m supérieur ou égal à 2) différents, reliés à des deuxièmes moyens de sélection 5b grâce auxquels un filtre donné peut être choisi, là encore en fonction du fluorophore que l'on souhaite analyser.

On choisit le nombre n de sources d'émission de lumière UV et le nombre m premiers de filtres passe-bande de telle sorte que la combinaison des n sources et des m filtres permet la mesure d'au moins 10 fluorophores différents, de préférence au moins 20 fluorophores différents, avantageusement au moins 30 fluorophores différents, typiquement au moins 40 fluorophores différents, le nombre de fluorophores étant égal à nxm.

Les longueurs d'onde correspondant à chaque premier filtre sont là encore typiquement comprises entre 300 et 700 nm.

Cet ensemble 4b peut notamment se présenter sous la forme d'une roue à filtres 40b, 41b, 4nb, mais il peut prendre d'autres formes, comme expliqué précédemment, du moment qu'il est possible de sélectionner de manière réversible le filtre au travers duquel on souhaite faire passer les émissions de fluorescence.

De façon générale, cet ensemble comporte au moins deux premiers filtres passe-bande différents et, de préférence, au moins douze filtres.

Le deuxième module 1b comporte un unique détecteur 6b d'émissions de fluorescence et une unité centrale 7b.

Le détecteur 6b est situé entre l'ensemble 4b de m premiers filtres et l'unité centrale 7b.

De préférence, un amplificateur 60b est prévu entre l'ensemble 4b et le détecteur 6b. Cet amplificateur permet d'augmenter la sensibilité de la sonde et donc la précision des mesures. Bien entendu, un tel amplificateur pourrait également être prévu dans la sonde illustrée à la figure 1.

En pratique, le détecteur 6b et l'amplificateur 60b sont regroupés dans un seul dispositif qui est un photomultiplicateur.

L'unité centrale comporte un microprocesseur 70b, un dispositif de pilotage 71b et une mémoire 72b, le dispositif 71b et la mémoire 72b étant contrôlés par le microprocesseur 70b, programmé de manière appropriée.

Comme l'illustre la figure 2, les premiers et deuxièmes moyens de sélection 3a et 5b sont reliés au dispositif de pilotage 71b qui les contrôle.

Par ailleurs, l'ensemble 4b et les premiers et deuxièmes moyens de sélection 3a et 5b sont reliés à une alimentation électrique 73b qui est contrôlée par l'unité centrale 7b.

En d'autres termes, comme dans la figure 1, l'unité centrale 7b alimente en électricité les différents composants de la sonde selon l'invention qui le nécessitent, et pilote leur fonctionnement.

Une liaison optique 91 est également prévue entre les moyens de collection optique 9 et l'ensemble 4b de filtres. Ces moyens de collection servent à recueillir et injecter dans la liaison 91 les photons émis par fluorescence. On peut également envisager de réaliser cette fonction directement avec la liaison optique. Cette liaison prend par exemple la forme d'une fibre optique dont la longueur est adaptée à l'utilisation. Cela permet de placer le premier module dans le milieu aquatique et de placer le deuxième module à une distance suffisante pour protéger les composants et, éventuellement, favoriser la transmission des résultats et/ou le réglage de la sonde.

Le fonctionnement de cette sonde 100 est similaire à celui qui a été décrit pour la sonde 1, à la différence que seul le premier module 1a est immergé dans le milieu aqueux 8 étudié.

L'unité centrale 7b commande, par l'intermédiaire du dispositif de pilotage 71b, les premiers moyens de sélection 3a pour sélectionner la source lumineuse émettant à la longueur d'onde souhaitée et les deuxièmes moyens de sélection 5b pour sélectionner le filtre passe-bande approprié, en fonction du fluorophore à analyser.

La source d'émission de lumière sélectionnée, par exemple la source 20a, émet alors de la lumière à travers le hublot 11a, vers le milieu aqueux 8. L'excitation lumineuse provenant de cette source est schématisée par la flèche F1.

Le fluorophore à analyser qui est présent dans le milieu aqueux 8 émet des émissions de fluorescence en direction du boîtier, en réponse à l'excitation lumineuse provenant de la source 20a sélectionnée. Les émissions de fluorescence, provenant du milieu 8 et traversant le hublot 11a, sont schématisées par la flèche F2.

Elles sont, dans un premier temps, collectées dans le premier module 1a, par les moyens de collection 9. On comprend que la présence de moyens de focalisation 90 permet d'optimiser la collection des émissions de fluorescence en provenance du milieu 8.

Les photons émis par fluorescence sont transmis par la liaison optique 91 au filtre de l'ensemble 4b qui a été sélectionné, lequel les filtre puis les transmet au détecteur 6b.

De préférence, ces photons filtrés sont amplifiés par l'amplificateur 60b avant d'être transmis au détecteur 6b qui les détecte et les convertit en un signal électrique représentatif de l'intensité de fluorescence.

Ce signal est ensuite traité par l'unité centrale 7b et, plus particulièrement par le microprocesseur 70b auquel le détecteur 6b est relié.

Ce traitement réalisé par le microprocesseur permet de déterminer la concentration du fluorophore en cause qui est présent dans le milieu 8, à partir d'une intensité de fluorescence.

Cette information peut être stockée dans la mémoire 72b. Elle peut également être transmise par le microprocesseur 70b vers un dispositif d'affichage à destination de l'utilisateur de la sonde. Cette transmission est schématisée par la flèche F3.

On comprend donc que cette sonde 100 présente les avantages de la sonde 1 illustrée à la figure 1, notamment ceux liés à la présence d'un unique détecteur, tout en étant plus robuste, les pièces les plus fragiles de la sonde étant placées dans un module qui peut rester à l'extérieur du milieu aqueux et de surcroît, immobile.

Cette conception permet l'utilisation d'un photomultiplicateur, qui présente donc l'avantage supplémentaire d'augmenter la sensibilité de la sonde, sans risquer un endommagement du photomultiplicateur.

On a pu ainsi constater que la sensibilité de cette sonde permet de détecter des concentrations beaucoup plus faibles que ce que permettent les sondes classiques.

Quelques exemples de sensibilité de détection vont maintenant être donnés :
- Tyrosine (mesurée au couple 275 nm /304 nm) : 1 µg.L⁻¹
- Tryptophane (mesurée au couple 275 nm /375 nm) : 0,1 µg.L⁻¹
- Acides humiques (mesurée au couple 365 nm/436 nm) : 0,1 mgC.L⁻¹

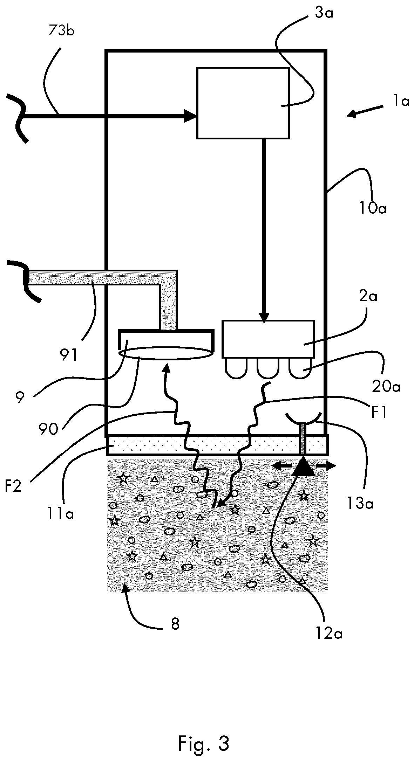

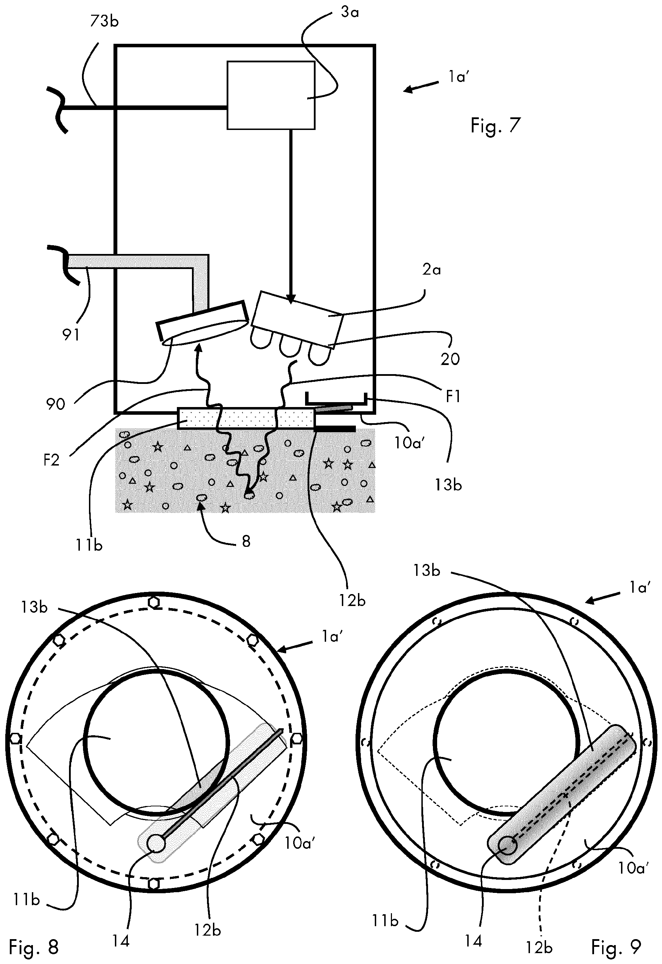

La figure 2 illustre également un système de nettoyage 12a-12b du hublot 11a-11b qui va être décrit plus en détail au regard des figures 3 à 12 qui n'illustrent que le premier module 1a-1a' selon une première version pour les figures 3 à 6, et selon une deuxième version pour les figures 7 à 12.

Ce système de nettoyage permet de nettoyer régulièrement le hublot pour empêcher la formation de biofilm sur celui-ci et il contribue donc au bon fonctionnement de la sonde.

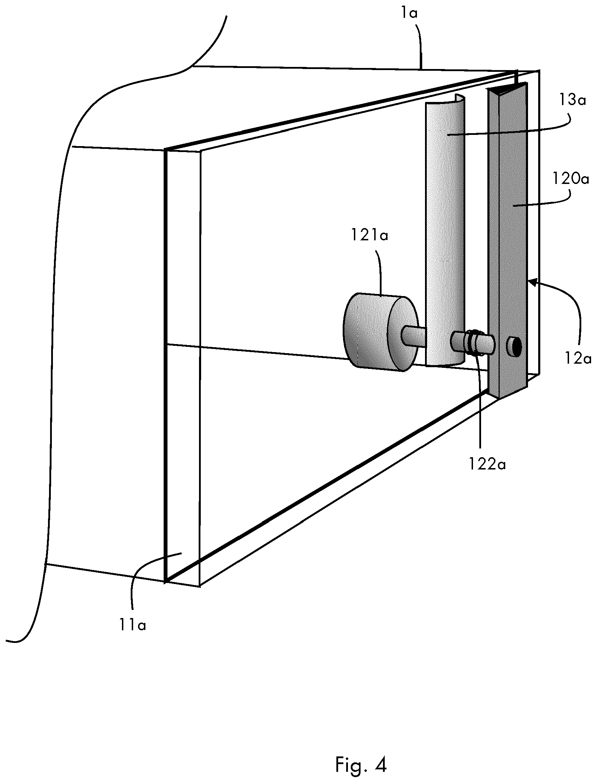

Comme l'illustre la figure 4, ce système de nettoyage 12a peut prendre la forme d'un essuie-glace 120a disposé à l'extérieur du premier module 1a et susceptible d'être actionné par un moteur 121a, disposé à l'intérieur du premier module 1a. L'essuie-glace est relié au moteur 121a par un axe 122a, cet axe entraînant l'essuie-glace en rotation lorsque le moteur fonctionne, de façon à effectuer un mouvement de va-et-vient sur le hublot.

Le système de nettoyage 12a est représenté en position escamotée sur les figures 3 et 4, le moteur étant alors à l'arrêt.

Les figures 3 et 4 montrent que le premier boîtier 1a comporte également une lame réfléchissante 13a qui est avantageusement montée sur l'axe 122a, à l'intérieur du boîtier 1a, entre les sources lumineuses de l'ensemble 2a et le hublot 11a, de préférence parallèlement et en regard de l'essuie-glace 120a.

Ainsi, la lame réfléchissante n'est pas soumise à la corrosion du milieu aqueux et les dépôts de matière provenant de ce milieu ou de biofilm sont également évités. Ses qualités de réflexion restent donc inchangées au cours de l'utilisation de la sonde.

Cette lame va permettre de contrôler et corriger la dérive dans le temps du fonctionnement des sources d'émission dans le domaine UV/visible 20a,21a, 2na ainsi que du détecteur 6b et de l'éventuel amplificateur 60b.

Dans la position escamotée des figures 3 et 4 du système de nettoyage 12a, la lame 13a est en position inactive, c'est-à-dire qu'elle ne se trouve pas sur le trajet des photons émis par la source lumineuse vers le milieu aqueux 8. Ainsi, dans le mode de réalisation des figures 3 et 4, la lame 13a est alors située le long d'un premier côté du hublot 11a.

Les figures 7 à 12 illustrent une deuxième version d'une sonde selon l'invention en deux modules séparés. Dans cette version, le module 1a' destiné à être immergé présente un hublot 11b de taille inférieure à la face 10a' du module qui le porte. Le système de nettoyage 12b et la lame 13b sont portés par un axe 14 porté par la face 10a', à côté du hublot 11b (sous le hublot sur les figures).

Dans la position escamotée du système de nettoyage 12b, la lame 13b est en position inactive. Dans le mode de réalisation des figures 7 à 9, la lame 13b est alors située en très grande partie en dehors du hublot 11b.

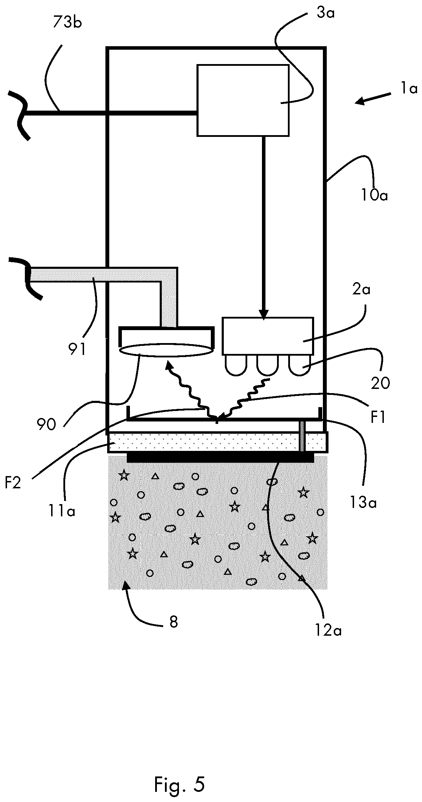

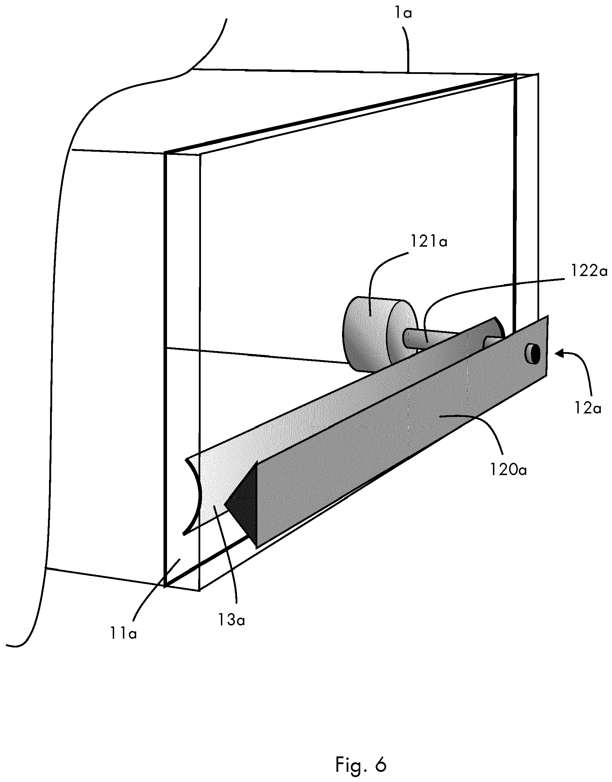

Il est maintenant fait référence aux figures 5 et 6 qui illustrent la lame 13a en position active dans la première version de la sonde selon l'invention. Comme l'illustre la figure 6, le système de nettoyage 12a, comme la lame 13a, sont positionnés sensiblement selon un deuxième côté du hublot 11a. Dans cette position, la lame 13a se trouve sur le trajet des photons émis par la source lumineuse vers le milieu aqueux 8, de sorte qu'elle peut intercepter la lumière émise par l'une des sources de l'ensemble 2a.

On comprend que, dans le mode de réalisation illustré, la lame 13a est placée dans cette position active par le moteur qui entraîne l'essuie-glace 120a ainsi que la lame 13a. Lorsque la lame est solidaire de l'essuie-glace, comme dans le mode de réalisation illustré, l'unité centrale est programmée pour permettre un arrêt du moteur en position active de la lame, de sorte que l'essuie-glace reste immobile tant que la mesure de dérive n'a pas été effectuée.

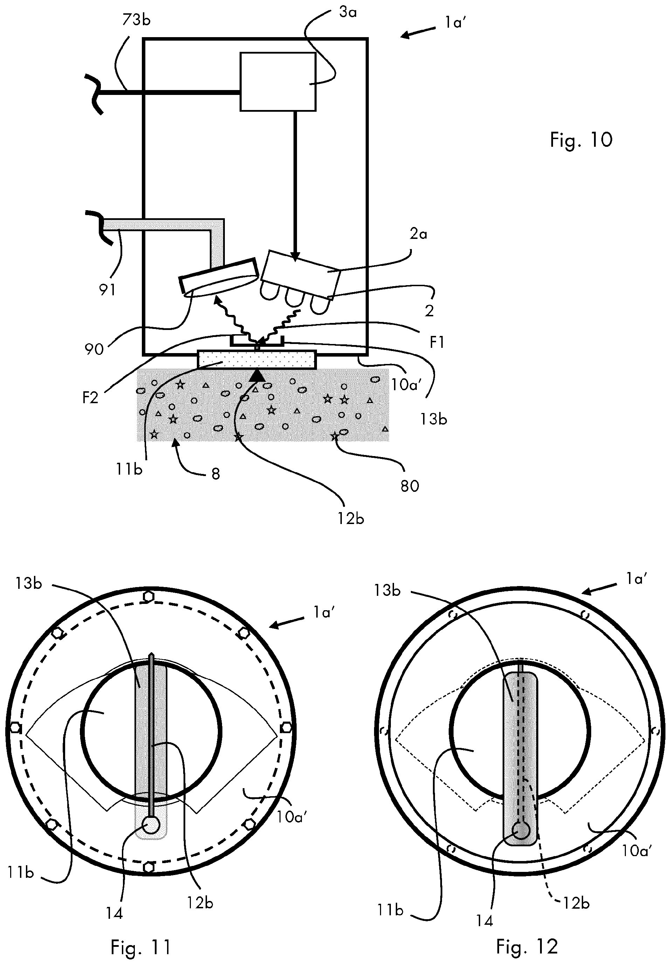

Les figures 10 à 12 illustrent la deuxième version d'une sonde selon l'invention avec un dispositif de contrôle de la dérive en position active.

Dans cette position, la lame 13b du dispositif de contrôle de la dérive se trouve sur le chemin de la majeure partie des photons émis par les sources d'excitation. Dans le mode de réalisation des figures 10 à 12, la lame 13b est alors située en très grande partie devant le hublot 11b, selon un diamètre du hublot de préférence.

Le contrôle de la dérive du fonctionnement d'une source d'émission de lumière UV/visible par exemple la source 20a, ainsi que du détecteur 6b et de l'éventuel amplificateur 60b est effectué de la façon suivante : la source 20a est sélectionnée par l'unité centrale au moyen des premiers moyens de sélection 3a.

Cette source 20a émet alors des photons à une longueur d'onde théorique connue, les photons sont réfléchis par la lame réfléchissante 13a en direction des moyens de collection 9.

L'unité centrale 7 commande alors les deuxièmes moyens de sélection 5b pour qu'ils sélectionnent le filtre de contrôle de la dérive qui est placé sur la roue à filtres, entre la lame réfléchissante et le détecteur. C'est un filtre à haute densité optique (OD 6) qui ne laisse donc passer que moins d'un millionième du flux de photons incidents. Un trou d'un diamètre de l'ordre du mm y a été percé en son milieu à l'aide d'une pointe, afin de laisser passer la lumière sans obstacle. Il s'agit donc d'un filtrage spatial.

Les photons collectés par les moyens de collection 9 sont transmis via la liaison optique 91 au filtre de contrôle de la dérive sélectionné et celui-ci les filtre et les transmet au détecteur 6b, via l'éventuel amplificateur 60b.

Le signal électrique issu du détecteur est traité par l'unité centrale 7b qui compare la puissance de ce signal à la puissance nominale de la source 20a qu'elle connaît. Ce traitement permet donc de déterminer si la source 20a émet bien des photons à sa puissance nominale.

Si la sonde selon l'invention comprend un filtre passe-bande en aval de chacune des n sources d'émission de lumière et en amont du hublot 11a, et si la source 20a dysfonctionne de telle sorte que la longueur d'onde émise soit différente de la longueur d'onde théorique d'émission de la source, alors, au cours de la mesure, le détecteur 6b ne recevra aucun signal ou un signal affaibli. L'unité centrale pourra donc en déduire que la source 20a ne fonctionne pas correctement et avertira l'utilisateur, en vue d'une maintenance.

Ainsi, ce dispositif de contrôle de la dérive permet de suivre l'évolution du signal mesuré dans le temps et d'identifier très simplement un glissement ou un dysfonctionnement des sources d'émission de photons, du détecteur et/ou de l'amplificateur, puisqu'il peut être actionné alors que la sonde est en cours d'utilisation dans un milieu aqueux. Par ailleurs, ce contrôle se fait à distance, in situ, sans avoir à intervenir physiquement sur le module.

En effet, la fluorescence du milieu aqueux est négligeable par rapport à la réflexion de la lumière par la lame. C'est pourquoi le dispositif est efficace même si la sonde est placée dans un milieu aqueux.

Par ailleurs, ce contrôle est réalisé par les moyens qui servent également à la mesure de la fluorescence. Ainsi, aucun canal optique supplémentaire n'est nécessaire pour réaliser ce contrôle, ce qui contribue à la compacité de la sonde et à la simplicité de sa conception.

Le contrôle peut être fait à la demande ou de manière systématique, pendant chaque nettoyage programmé par l'unité centrale.

Si des défauts sont constatés, un échange de pièces peut être réalisé lorsque le fonctionnement est erratique, ou un recalibrage des calculs peut être effectué lorsque l'on connaît précisément la dérive et que celle-ci évolue lentement, mais de manière constante (jusqu'au remplacement in fine des composants concernés bien entendu).

L'invention n'est cependant pas limitée à ce mode de réalisation. Ainsi, le passage de la lame de sa position inactive à sa position active, et vice-versa, pourrait également être commandé par l'unité centrale, indépendamment du nettoyage du hublot.

Bien entendu, un système de nettoyage et un dispositif de contrôle de la dérive peuvent être aussi prévus dans la sonde 1 illustrée à la figure 1.

En outre, le système de nettoyage et le dispositif de contrôle de la dérive peuvent être indépendants l'un de l'autre.

## Revendications

1. Sonde optique destinée à être, en utilisation, au moins partiellement immergée dans un milieu aqueux (8) comprenant des molécules organiques présentant au moins un fluorophore, comprenant :
- Un ensemble (2, 2a) de n sources (20, 21, 2n ;20a, 21a, 2na) d'émission de lumière UV/visible à des longueurs d'onde différentes, n étant un entier supérieur ou égal à 2, lesdites sources étant agencées pour diriger de la lumière vers l'extérieur de la sonde et dans le milieu aqueux lorsque la sonde est en utilisation, lesdites sources étant associées à des premiers moyens de sélection (3, 3a),
- Un ensemble (4, 4a) de m premiers filtres passe-bande différents (40, 41, 4n ;40b, 41b, 4nb), m étant un entier supérieur ou égal à 2, associés à des deuxièmes moyens de sélection (5, 5b), agencé pour recevoir des émissions de fluorescence émises par ledit au moins un fluorophore présent dans le milieu aqueux en réponse à une excitation lumineuse provenant de l'une desdites sources d'émission de lumière ;
- Un détecteur (6, 6b) d'émissions de fluorescence filtrées par un premier filtre sélectionné par lesdits deuxièmes moyens de sélection associés audit ensemble de m premiers filtres et transmises par ledit ensemble (40, 41, 4n ;40b, 41b, 4nb) pour convertir en un signal électrique lesdites émissions et
- Une unité centrale (7,7b) conçue pour contrôler les premiers et deuxièmes moyens de sélection et pour déterminer la concentration dudit au moins un fluorophore, à partir dudit signal électrique,
**caractérisée en ce que** la sonde comprend un premier module (1a) et un deuxième module (1b) reliés entre eux par des moyens de connexion électriques (73b) et optiques (91), le premier module (1a) regroupant l'ensemble (2a) des sources d'émission de lumière, les premiers moyens de sélection (3a) et des moyens de collection optique (9) conçus pour collecter des émissions de fluorescence émises par le milieu aqueux en réponse à l'excitation lumineuse provenant de l'une des dites sources d'émission de lumière et les transmettre au deuxième module (1b) par les moyens de connexion optiques (91), et le deuxième module (1b) regroupant l'ensemble (4b) de filtres passe-bande, les deuxièmes moyens de sélection (5b), le détecteur (6b) et l'unité centrale (7b), le premier module étant destiné, en utilisation, à être immergé dans le milieu aqueux.

2. Sonde selon la revendication 1 , comprenant un amplificateur associé au détecteur (6, 6b).

3. Sonde selon l'une quelconque des revendications 1 ou 2, dans laquelle un deuxième filtre passe-bande (50) est placé en aval de chacune des n sources d'émission de lumière.

4. Sonde selon l'une quelconque des revendications 1 à 3, dans laquelle des moyens de focalisation (90) sont prévus en amont des moyens de collection optique.

5. Sonde selon l'une quelconque des revendications 1 à 4, dans laquelle les deuxièmes moyens de sélection (4, 4b) consistent en une roue à filtres.

6. Sonde selon l'une quelconque des revendications 1 à 5, dans laquelle l'unité centrale (7, 7b) comprend un microprocesseur (70, 70b), un dispositif de pilotage (71, 71b) et une mémoire (72, 72b).

7. Sonde selon l'une quelconque des revendications 1 à 6 comprenant un dispositif de contrôle de la dérive du fonctionnement des sources d'émission de lumière, du détecteur et de l'éventuel amplificateur.

8. Sonde selon l'une quelconque des revendications 1 à 7 comprenant un boîtier (1, 1a) dont une partie (11, 1 1a) formant hublot est transparente pour le passage de la lumière émise par les sources et des émissions de fluorescence émises par ledit au moins un fluorophore, en utilisation, dans le domaine UV/visible.

9. Sonde selon la revendication 8 comprenant un système de nettoyage associé audit hublot.

10. Sonde selon l'une quelconque des revendications 8 à 9, dans laquelle le dispositif de contrôle de la dérive du fonctionnement des sources d'émission de lumière comporte une lame réfléchissante (13a).

11. Sonde selon la revendication 10, dans laquelle la lame réfléchissante (13a) est associée au système de nettoyage.

12. Procédé de mesure de la concentration d'au moins un fluorophore dans un milieu aqueux (8) comprenant des molécules organiques présentant au moins un fluorophore, le procédé utilisant la sonde selon l'une quelconque des revendications 1 à 11 et comprenant les étapes suivantes :
- La sélection d'une source d'émission de lumière parmi l'ensemble (2,2a) des n sources d'émission de lumière à des longueurs d'onde différentes, dans la gamme 250-600 nm ;
- L'émission de lumière à travers ledit milieu aqueux à partir de la source sélectionnée ;
- La sélection d'un filtre passe-bande parmi l'ensemble des m premiers filtres ;
- La filtration et la détection des émissions de fluorescence émises par le milieu aqueux en réponse à l'excitation lumineuse provenant de ladite source sélectionnée ;
- Le traitement des émissions de fluorescence filtrées et détectées afin de déterminer la concentration dudit au moins un fluorophore.

13. Procédé selon la revendication 12, dans lequel la filtration des émissions de fluorescence est réalisée avant leur détection.

14. Procédé selon la revendication 12 ou 13, dans lequel les émissions de fluorescence filtrées passent à travers un amplificateur avant d'être détectées et traitées.

## Patentansprüche

1. Optischer Sensor, dazu eingerichtet, im Gebrauch zumindest teilweise in ein wässriges Medium (8) eingetaucht zu werden, das organische Moleküle mit mindestens einem Fluorophor enthält, umfassend:
eine Anordnung (2, 2a) von n Quellen (20, 21, 2n; 20a, 21a, 2na) zur Emission von UV/sichtbarem Licht bei unterschiedlichen Wellenlängen, wobei n eine Ganzzahl größer oder gleich 2 ist, wobei die Quellen so angeordnet sind, Licht nach außen aus dem Sensor und in das wässrige Medium zu richten, wenn der Sensor in Gebrauch ist, wobei die Quellen ersten Auswahlmitteln (3, 3a) zugeordnet sind,
eine Anordnung (4, 4a) von m verschiedenen ersten Bandpassfiltern (40, 41, 4n; 40b, 41b, 4nb), wobei m eine Ganzzahl höher oder gleich 2 ist, die zweiten Auswahlmitteln (5, 5b) zugeordnet sind, dafür angeordnet, Fluoreszenzemissionen zu empfangen, die von dem mindestens einen in dem wässrigen Medium vorhandenen Fluorophor als Reaktion auf eine Lichtanregung aus einer der Lichtemissionsquellen ausgestrahlt werden;
einen Detektor (6, 6b) für Fluoreszenzemissionen, die durch ein erstes Filter gefiltert werden, das durch die zweiten Auswahlmittel ausgewählt wird, welche der Anordnung von m ersten Filtern zugeordnet sind und durch die Anordnung (40, 41, 4n; 40b, 41b, 4nb) übertragen werden, um die Emissionen in ein elektrisches Signal umzuwandeln, und
eine Zentraleinheit (7, 7b), die zur Steuerung der ersten und der zweiten Auswahlmittel sowie zur Bestimmung der Konzentration des wenigstens einen Fluorophors von dem elektrischen Signal aus ausgebildet ist,
**dadurch gekennzeichnet,**
**dass** der Sensor ein erstes Modul (1a) und ein zweites Modul (1b) umfasst, die durch elektrische (73b) und optische (91) Verbindungsmittel miteinander verbunden sind, wobei das erste Modul (1a) die Anordnung (2a) der Lichtemissionsquellen, die ersten Auswahlmittel (3a) und optische Sammelmittel (9) zusammenfasst, welche dazu ausgebildet sind, Fluoreszenzemissionen zu sammeln, die durch das wässrige Medium als Reaktion auf die Lichtanregung aus einer der Lichtemissionsquellen ausgestrahlt werden, und sie über die optischen Verbindungsmittel (91) an das zweite Modul (1b) zu übertragen, und wobei das zweite Modul (1b) die Anordnung (4b) von Bandpassfiltern, die zweiten Auswahlmittel (5b), den Detektor (6b) und die Zentraleinheit (7b) zusammenfasst, wobei das erste Modul dazu eingerichtet ist, in Gebrauch in das wässrige Medium eingetaucht zu werden.

2. Sensor nach dem Anspruch 1,
mit einem Verstärker, der dem Detektor (6, 6b) zugeordnet ist.

3. Sensor nach einem der Ansprüche 1 oder 2,
wobei ein zweites Bandpassfilter (50) vor jeder der n Lichtemissionsquellen angeordnet ist.

4. Sensor nach einem der Ansprüche 1 bis 3,
wobei Fokussierungsmittel (90) den optischen Sammelmitteln vorgelagert sind.

5. Sensor nach einem der Ansprüche 1 bis 4,
wobei die zweiten Auswahlmittel (4, 4b) aus einem Filterrad bestehen.

6. Sensor nach einem der Ansprüche 1 bis 5,
wobei die Zentraleinheit (7, 7b) einen Mikroprozessor (70, 70b), eine Treibereinrichtung (71, 71b) und einen Speicher (72, 72b) umfasst.

7. Sensor nach einem der Ansprüche 1 bis 6,
eine Einrichtung zur Steuerung der Fehlfunktion der Lichtemissionsquellen, des Detektors und des möglichen Verstärkers umfassend.

8. Sensor nach einem der Ansprüche 1 bis 7,
ein Gehäuse (1, 1a) umfassend, von dem ein Fensterteil (11, 11a) für das Licht durchlässig ist, welches durch die Quellen ausgestrahlt wird, und für Fluoreszenzemissionen, welche durch den wenigstens einen Fluorophor im Gebrauch im UV-/sichtbaren Bereich ausgestrahlt werden.

9. Sensor nach Anspruch 8,
ein Reinigungssystem umfassend, das dem Fensterteil zugeordnet ist.

10. Sensor nach einem der Ansprüche 8 bis 9,
wobei die Einrichtung zur Steuerung der Fehlfunktion der Lichtemissionsquellen ein reflektierendes Paneel (13a) umfasst.

11. Sensor nach Anspruch 10,
wobei das reflektierende Paneel (13a) dem Reinigungssystem zugeordnet ist.

12. Verfahren zur Messung der Konzentration wenigstens eines Fluorophors in einem wässrigen Medium (8), organische Moleküle umfassend, die wenigstens einen Fluorophor aufweisen,
wobei das Verfahren den Sensor nach einem der Ansprüche 1 bis 11 verwendet und die folgenden Schritte umfasst:
die Auswahl einer Lichtemissionsquelle aus der Anordnung (2, 2a) der n Lichtemissionsquellen bei unterschiedlichen Wellenlängen im Bereich 250-600 nm;
die Emission von Licht durch das wässrige Medium hindurch aus der gewählten Quelle;
die Auswahl eines Bandpassfilters aus der Anordnung der m ersten Filter;
das Filtern und die Erkennung der Fluoreszenzemissionen, die durch das wässrige Medium als Reaktion auf die Lichtanregung aus der ausgewählten Quelle ausgestrahlt werden;
die Behandlung der Fluoreszenzemissionen, die zur Bestimmung der Konzentration des wenigstens einen Fluorophors gefiltert und erkannt werden.

13. Verfahren nach Anspruch 12,
wobei das Filtern der Fluoreszenzemissionen erfolgt, bevor sie erkannt werden.

14. Verfahren nach Anspruch 12 oder 13,
wobei die gefilterten Fluoreszenzemissionen durch einen Verstärker laufen, bevor sie erkannt und behandelt werden.

## Claims

1. Optical probe that is intended, in use, to be at least partially immersed in an aqueous medium (8) comprising organic molecules including at least one fluorophore, comprising:
A set (2, 2a) of n sources (20, 21, 2n; 20a, 21a, 2na) of emission of UV/visible light at different wavelengths, n being an integer greater than or equal to 2, said sources being arranged to direct light towards the outside of the probe and in the aqueous medium when the probe is in use, said sources being associated with first selection means (3, 3a),
A set (4, 4a) of m different first bandpass filters (40, 41, 4n; 40b, 41b, 4nb), m being an integer greater than or equal to 2, associated with second selection means (5, 5b), arranged to receive fluorescence emissions emitted by said at least one fluorophore present in the aqueous medium in response to a light excitation originating from one of said light emission sources;
A detector (6, 6b) of fluorescence emissions filtered by a first filter selected by said second selection means associated with said set of m first filters and transmitted by said set (40, 41, 4n; 40b, 41b, 4nb) to convert said emissions into an electrical signal and
A central unit (7, 7b) designed to control the first and second selection means and to determine the concentration of said at least one fluorophore, from said electrical signal,
**characterized in that** the probe comprises a first module (1a) and a second module (1b) linked to one another by electrical (73b) and optical (91) connection means, the first module (1a) combining the set (2a) of the light emission sources, the first selection means (3a) and optical collection means (9) designed to collect fluorescence emissions emitted by the aqueous medium in response to the light excitation originating from one of said light emission sources and transmit them to the second module (1b) by the optical connection means (91), and the second module (1b) combining the set (4b) of bandpass filters, the second selection means (5b), the detector (6b) and the central unit (7b), the first module being intended, in use, to be immersed in the aqueous medium.

2. Probe according to Claim 1, comprising an amplifier associated with the detector (6, 6b).

3. Probe according to either one of Claims 1 and 2, wherein a second bandpass filter (50) is placed downstream of each of the n light emission sources.

4. Probe according to any one of Claims 1 to 3, wherein focusing means (90) are provided upstream of the optical collection means.

5. Probe according to any one of Claims 1 to 4, wherein the second selection means (4, 4b) consist of a filter wheel.

6. Probe according to any one of Claims 1 to 5, wherein the central unit (7, 7b) comprises a microprocessor (70, 70b), a driver device (71, 71b) and a memory (72, 72b).

7. Probe according to any one of Claims 1 to 6, comprising a device for controlling the drift of operation of the light emission sources, of the detector and of the possible amplifier.

8. Probe according to any one of Claims 1 to 7, comprising a housing (1, 1a) of which a window-forming part (11, 11a) is transparent for the passage of the light emitted by the sources and of the fluorescence emissions emitted by said at least one fluorophore, in use, in the UV/visible range.

9. Probe according to Claim 8, comprising a cleaning system associated with said window.

10. Probe according to either one of Claims 8 and 9, wherein the device for controlling the drift of operation of the light emission sources comprises a reflecting plate (13a).

11. Probe according to Claim 10, wherein the reflecting plate (13a) is associated with the cleaning system.

12. Method for measuring the concentration of at least one fluorophore in an aqueous medium (8) comprising organic molecules including at least one fluorophore, the method using the probe according to any one of Claims 1 to 11 and comprising the following steps:
The selection of a light emission source from among the set (2, 2a) of the n light emission sources at different wavelengths, in the 250-600 nm range;
The emission of light through said aqueous medium from the selected source;
The selection of a bandpass filter from among the set of the m first filters;
The filtration and detection of the fluorescence emissions emitted by the aqueous medium in response to the light excitation originating from said selected source;
The processing of the filtered and detected fluorescence emissions in order to determine the concentration of said at least one fluorophore.

13. Method according to Claim 12, wherein the filtration of the fluorescence emissions is performed before the detection thereof.

14. Method according to Claim 12 or 13, wherein the filtered fluorescence emissions pass through an amplifier before being detected and processed.
